Europäisches Patentamt

European Patent Office

Office européen des brevets

(19)

(11) Veröffentlichungsnummer : **0 062 310**
**B1**

(12) **EUROPÄISCHE PATENTSCHRIFT**

(45) Veröffentlichungstag der Patentschrift :
**11.12.85**

(21) Anmeldenummer : **82102773.7**

(22) Anmeldetag : **01.04.82**

(51) Int. Cl.⁴ : **C 12 Q   1/56**, G 01 N 33/86

(54) **Reagens zur optischen Bestimmung des Blutgerinnungsverhaltens.**

(30) Priorität : **02.04.81 DE 3113350**

(43) Veröffentlichungstag der Anmeldung :
**13.10.82 Patentblatt 82/41**

(45) Bekanntmachung des Hinweises auf die Patenterteilung : **11.12.85 Patentblatt 85/50**

(84) Benannte Vertragsstaaten :
**AT BE CH DE FR GB IT LI LU NL SE**

(56) Entgegenhaltungen :
**EP-A- 0 014 039**
**CHEMICAL ABSTRACTS, Band 83, Nr. 18, 3. Novem-**
**ber 1975, Seite 99, Nr. 158537d, Columbus, Ohio,**
**USA, P.M. BAYER et al.: "Solubilization of tissue**
**thromboplastin. Effect of various detergents"**
**CHEMICAL ABSTRACTS, Band 77, Nr. 7, 14. August**
**1972, Seiten 210, 211, Nr. 45185y, Columbus, Ohio,**
**USA; YIN-SHAN SHUM et al.: " Thromboplastic acti-**
**vity of acetone-dehydrated chicken brain powder**
**extracts after repeated extraction and dilution"**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Jering, Helmut, Dr.**
**Apolloweg 5**
**D-8000 München (DE)**
Erfinder : **Becker, Udo, Dr.**
**Schmaedelstrasse 17**
**D-8000 München 60 (DE)**
Erfinder : **Roeschlau, Peter, Dr.**
**Schönegertstrasse 4**
**D-8124 Seeshaupt (DE)**

(74) Vertreter : **Weickmann, Heinrich, Dipl.-Ing. et al**
**Patentanwälte Dipl.-Ing. H.Weickmann Dipl.-Phys.Dr.**
**K.Fincke Dipl.-Ing. F.A.Weickmann Dipl.-Chem. B.**
**Huber Dr.-Ing. H. Liska Dipl.-Phys.Dr. J. Prechtel**
**Postfach 860820**
**D-8000 München 86 (DE)**

## Beschreibung

Die Erfindung betrifft ein Reagens zur optischen Bestimmung des Blutgerinnungsverhaltens des sogenannten exogenen Gerinnungssystems (Quick Test).

Gerinnungsphysiologische Untersuchungen finden in Krankenhäusern, Arztpraxen und Laboratorien in breitem Umfang Anwendung u. a. zur präoperativen Untersuchung, zur Erkennung eines Blutungs- oder Thrombose-Risikos, zur Überwachung der Antikoagulantien-Therapie bei thrombosegefährdeten Patienten und bei der Verlaufskontrolle zahlreicher Erkrankungen, z. B. schwere Infektionen, Leberfunktionsschäden und maligne Erkrankungen.

Die am häufigsten durchgeführten Gerinnungstests sind Globaltests, bei denen gleichzeitig die Funktion mehrerer der am Gerinnungsvorgang beteiligten Komponenten bestimmt wird. Als wichtigste Global-Tests gelten der sog. Prothrombin-Zeittest (Quick-Test, A. J. Quick, Am. J. Physiol. 118, 260 (1937) und der sog. aktivierte partielle Thromboplastin-Zeittest (PTT). Im folgenden wird unter « Quick-Test » die Bestimmung des Blutgerinnungsverhaltens des exogenen Gerinnungssystems verstanden. Dieses System wird durch die Freisetzung von Thromboplastin aus beschädigtem Gewebe initiiert. Der Quick-Test gibt Auskunft über den Funktionszustand der dem sog. exogenen Gerinnungssystem zuzurechnenden Komponenten, der PTT-Test läßt den Funktionszustand der am endogenen Gerinnungssystem beteiligten Komponenten erkennen, welches durch Kontaktaktivierung von Faktor XII ausgelöst wird. Bei pathologischem Ausfall der Tests kann der Defekt zunächst keiner bestimmten Komponente zugeordnet werden. Dies muß durch eine anschließende Bestimmung von einzelnen Gerinnungsfaktoren erfolgen, wofür ebenfalls geeignete Testsysteme bekannt sind.

Bei der Durchführung gerinnungsphysiologischer Tests ist die übliche Meßgröße zumeist die Gerinnungszeit. Es wird dabei die Zeit registriert, die nach Zugabe des Reagens zur Untersuchungsflüssigkeit bis zur Bildung eines Gerinnsels vergeht. Unabhängig von der Art der zu bestimmenden Gerinnungsfaktoren sind die bekannten Tests so angelegt, daß letztlich die Überführung von Fibrinogen in Fibrin durch das Enzym Thrombin ausgelöst wird. Die Umwandlung von Fibrinogen (löslich) in Fibrin (unlöslich) dient daher als Indikatorreaktion, außer wenn Fibrinogen selbst bestimmt werden soll.

Für die Messung des Gerinnungseintritts sind zahlreiche Methoden bekannt. Am häufigsten wird in den Gerinnungsansatz periodisch ein Platinhäkchen eingeführt, welches bei der Bildung eines Gerinnsels einen Fibrinfaden aus der Lösung herauszieht. Dieser Zeitpunkt wird dann als Gerinnungsendpunkt registriert. Der Registrierungsvorgang kann manuell mittels Stoppuhr oder elektrisch durch Auslösung eines Kontakts, wenn das Platinhäkchen als Elektrode ausgelegt ist, erfolgen. Eine Automation im Sinne üblicher klinisch-chemischer Analysen ist nach diesem System nicht möglich, da kein kontinuierlicher Durchsatz von Proben möglich ist, die Gerinnsel nicht automatisch ausgespült werden können und die häkchen zwischen den Analysen nicht ausreichend gereinigt werden können.

Eine andere Möglichkeit zur Registrierung von Gerinnungszeiten stellt die bei der Gerinnselbildung auftretende Trübung dar, die durch die Bildung des unlöslichen Fibrins hervorgerufen wird. Es sind optisch registrierende Koagulometer bekannt, die nach diesem Prinzip arbeiten. In der Praxis sind diesem Prinzip aber Grenzen gesetzt, da viele Plasmen eine hohe Eigentrübung aufweisen, die verwendeten Reagentien z. T. selbst trübe sind und bei pathologischem Untersuchungsmaterial die Gerinnung so langsam erfolgen kann, daß kein eindeutiges Signal entsteht.

Die einfachste Möglichkeit, Trübungsprobleme zu vermeiden, wäre, bei höherer Verdünnung zu arbeiten. Dies ist jedoch bei dem bekannten Verfahren nicht ohne weiteres möglich, da gleichzeitig mit der Verdünnung auch die Reaktionszeit der Gerinnungstests stark verlängert wird und der mögliche Vorteil der Automation durch verlängerte Reaktionszeiten wieder aufgehoben wird. Von einer bestimmten Verdünnung an reicht auch das in der Probe enthaltene Fibrinogen nicht mehr für die Indikatorreaktion, welche in der Umwandlung von Fibrinogen in Fibrin besteht, aus. 1 : 10 verdünntes Plasma hat z. B. gegenüber unverdünntem Plasma im Quick-Test eine ca. 3-5-fach verlängerte Gerinnungszeit. Das Ausmaß der Verlängerung von Gerinnungszeiten durch Verdünnung wurde quantitativ u. a. von Biggs, Proceedings of the Royal Society (GB) 173, 421-441 (1969) und Girolami, Blut 28, 351-360 (1974) beschrieben.

Besonders stark verlängerte Reaktionszeiten werden erhalten, wenn über die Probenverdünnung hinaus noch zusätzlich die Reagentien verdünnt werden (s. Biggs). Eine Reagentien-Verdünnung ist jedoch ebenfalls als Vorbedingung für eine von Trübungsproblemen freie optische Messung von Gerinnungsvorgängen anzusehen. Die in der Gerinnungsdiagnostik eingesetzten Reagentien besitzen nämlich eine mit ihrer Funktion unmittelbar verbundene Eigentrübung. Die für die Durchführung des Quick-Tests verwendeten Gewebsthromboplastine sind milchig trübe Suspensionen aus Säugetiergewebe, z. B. Kaninchenhirn.

Es wurden schon Methoden und Reagentien beschrieben, die für die Durchführung globaler Gerinnungstests in verdünntem Medium geeignet sind. Sie beruhen auf der Verwendung synthetischer chromogener Peptidsubstrate zur Durchführung der Indikatorreaktion, da, wie erwähnt, bei Vorverdünnung der Fibrinogengehalt der Probe nicht mehr ausreicht. Die synthetischen chromogenen Substrate treten dabei an die Stelle des natürlichen Substrats, Fibrinogen. Das optische Signal entsteht bei der Spaltung des Substrats durch Thrombin, welches ein optisch bestimmbares Fragment freisetzt

0 062 310

(vergleiche Paulsen et al Clinica Chimica Acta 92, 465-468 (1979), Yamada et al Thrombosis Research 15, 351-358 (1979) und EP-OS Nr. 0014039).

Ein Nachteil des Verfahrens unter Verwendung eines synthetischen Substrats und von Thromboplastin für den optischen Quick-Test besteht darin, daß es nicht möglich ist, ein vorgemischtes 1-Komponenten-Reagens zu beschaffen, welches sämtliche für die Durchführung des Tests erforderlichen Substanzen enthält. Thromboplastin weist nämlich eine proteolytische Wirksamkeit auf, welche zu einer Spaltung des Substrats führt und damit die Substratspaltung durch das gebildete Thrombin verfälscht. Außerdem würde ein derartiges Reagenz auch nicht lagerfähig sein.

Der Erfindung liegt die Aufgabe zugrunde, dieses Problem zu lösen und ein Reagenz zu schaffen, welches sämtliche für die Durchführung des Tests erforderlichen Substanzen vorgemischt enthält, lagerfähig ist und richtige Ergebnisse liefert.

Erfindungsgemäß gelingt dies durch ein Reagenz zur optischen Bestimmung des Blutgerinnungsverhaltens (Quick-Test), enthaltend Thromboplastin, ein synthetisches Thrombinsubstrat und Puffer, das durch einen Gehalt an nicht proteolytisch wirksamem Thromboplastin gekennzeichnet ist, welches durch Herstellen eines Acetontrockenpulvers aus Hirn, Inkubation einer neutralen wäßrigen Lösung des Acetontrockenpulvers bei 25 bis 40 °C, Gewinnung der unlöslichen Fraktion, Behandlung einer Suspension dieser Fraktion in verdünnter Salzlösung mit einem oberflächenaktiven Mittel in Gegenwart von Formiat und Trocknung der abgetrennten löslichen Fraktion erhältlich ist.

Jede Herstellung eines Acetontrockenpulvers aus dem Gewebsmaterial kann nach für die Acetontrockenpulverherstellung bekannten Methoden erfolgen. Ein geeignetes Verfahren ist beispielsweise R. Biggs, « Human Blood Coagulation, Haemostatis and Thrombosis », Blackwell Scientific Publication Oxford 1976, Seite 663 beschrieben.

Das Acetontrockenpulver wird in einer neutralen wäßrigen Lösung suspendiert und unter Rühren bei der angegebenen Temperatur inkubiert. Bevorzugt wird eine Temperatur zwischen 35 und 40 °C. Unter diesen Bedingungen dauert die Inkubation etwa eine halbe Stunde. Der pH-Wert kann dabei zwischen 6,0 und 8,0, vorzugsweise 6,7 bis 7,3, liegen. Zweckmäßig wird er durch einen geeigneten Puffer konstant gehalten. Die unlösliche Fraktion läßt sich durch Zentrifugieren, beispielsweise bei 2 500 g, abtrennen. Der bei der Zentrifugation erhaltene Niederschlag wird erneut in einer verdünnten Salzlösung suspendiert und vorzugsweise mit einem oberflächenaktiven Mittel der Cholsäuregruppe in Gegenwart von Formiat behandelt. Für die Salzlösung wird Kochsalz bevorzugt. Geeignete Konzentrationen liegen zwischen 0,2 und 2, vorzugsweise 0,5 bis 1 %. Die Konzentration an oberflächenaktiven Mitteln liegt vorzugsweise zwischen 0,01 und 0,2 %, insbesondere wenn die bevorzugten Cholate verwendet werden. Es können jedoch auch andere oberflächenaktive Mittel verwendet werden.

Als Formiat kommen die Alkali- und Erdalkaliformiate in erster Linie in Betracht. Besonders bevorzugt wird Calciumformiat. Die Konzentration liegt vorzugsweise zwischen 0,005 und 0,05 Mol/l. Die Behandlung wird bevorzugt ebenfalls im Temperaturbereich zwischen 25 und 40 °C durchgeführt, die Zeitdauer entspricht der der Inkubation des Trockenpulvers. Schließlich wird abzentrifugiert, beispielsweise bei 2 500 x g, und die erhaltene Suspension eines nicht mehr proteolytisch wirksamen Thromboplastins, ggf. nach vorherigem Zusatz der anderen Bestandteile des erfindungsgemäßen Reagens, gefriergetrocknet. Das Reagens kann jedoch auch aus den festen trockenen Substanzen in den gewünschten Mengenverhältnissen zusammengemischt werden.

Als synthetisches Thrombinsubstrat kann prinzipiell jedes der bekannten synthetischen Thrombinsubstrate verwendet werden. Zahlreiche derartige Thrombinsubstrate sind handelsüblich. Bevorzugt wird das Substrat Tos—Gly—Pro—Arg—pNA sowie das unter der Bezeichnung S-2238 vertriebene H—D—Phe—Pip—Arg—pNA. Diese Substrate liefern bei der enzymatischen Spaltung gefärbte Spaltprodukte, die sich leicht optisch quantitativ bestimmen lassen.

Das erfindungsgemäße Reagens enthält Thromboplastin in einer Konzentration von 3 bis 20 Vol-%, vorzugsweise 6 bis 10 Vol-%, mit einer Aktivität wie in Beispiel 1 beschrieben. Synthetisches Thrombinsubstrat in einer Menge von $2 \times 10^{-5}$ bis $20 \times 10^{-5}$ Mol/l sowie Puffer pH, 7,2 bis 8,5. Die Pufferkonzentration liegt vorzugsweise zwischen 0,05 und 0,25 Mol/l. Im Prinzip ist jeder im angegebenen pH-Bereich wirksame Puffer geeignet, besonders bevorzugt wird jedoch Trispuffer. Die besten Ergebnisse werden mit 0,1 Mol/l Puffer pH 8,1 erhalten. Die besonders bevorzugte Substratkonzentration liegt um $5 \times 10^{-5}$ Mol/l.

Vorzugsweise enthält das erfindungsgemäße Reagens außerdem Ca-Salz in einer Konzentration von 2 bis 10 mMol/l, wie die obigen Angaben, jeweils bezogen auf fertige Reagenslösung.

Als Ca-Salz eignet sich jedes leicht wasserlösliche Ca-Salz, beispielsweise ein Halogenid, Acetat oder dergleichen. Bevorzugt wird Calciumchlorid.

Vorzugsweise enthält das Reagens außerdem noch 0,5 bis 3 Gew.-% Harnstoff. Besonders bevorzugt sind 1,4 bis 1,6 %.

Ein erfindungsgemäßes Reagens in der obigen Zusammensetzung stellt zwar in Form der gebrauchsfertigen Lösung ein verdünntes System dar, die Reaktionsdauer gegenüber den bekannten Tests im unverdünnten System ist jedoch nur unwesentlich verlängert. Die Bestimmung dauert bei Normalplasma etwa 30 sec, bei Plasmen von Personen, die unter Thrombose-Prophylaxe mittels oraler Antikoagulantien stehen, etwa 60 bis 120 sec. Bei den bekannten unverdünnten Quick-Tests mit den oben geschilderten Nachteilen liegen die entsprechenden Zeiten für Normalplasma bei ca. 15 sec, bei den genannten

3

pathologischen Plasmen um 30 bis 60 sec. Demgegenüber benötigen die bekannten, hinsichtlich der Verdünnung mit dem erfindungsgemäßen Reagens vergleichbaren Tests etwa die fünffache Reaktionszeit.

Das erfindungsgemäße Reagens wird zweckmäßig so angewendet, daß die Zeit bis zur Erreichung einer bestimmten Absorption bei gegebener Wellenlänge gemessen wird. Es ist jedoch auch eine kinetische Auswertung möglich, wobei dann zweckmäßig eine Eichkurve der Auswertung zugrundegelegt wird. Die gegenüber bekannten optischen Quick-Tests auf der Basis synthetischer Substrate wesentlich verringerte Bestimmungsdauer wird auf eine synergistische Wirksamkeit der außerhalb der physiologischen Bereiche liegenden Ionenstärke und pH-Werte und der unter der $K_M$ liegenden Substratkonzentration zurückgeführt.

Die folgenden Beispiele erläutern die Erfindung weiter :

### Beispiel 1

A) Herstellung von nicht proteolytischem Thromboplastin : Aus frisch gewonnenem Kaninchenhirn wird durch Acetonbehandlung ein Trockenpulver nach dem Verfahren von R. Biggs, « Human Blood Coagulation, Haemostatis and Thrombosis », Blackwell Scientific Publications Oxford 1976, S. 663, hergestellt und bis zur Gewichtskonstanz getrocknet.

2,5 g obigen Pulvers werden in 50 ml 0,1 Mol/l Natriumacetat-Puffer, pH 7, suspendiert und 30 min lang bei 37 °C gerührt. Danach wird 15 min lang bei 2 500 g zentrifugiert und der Niederschlag in 50 ml 0,7 %iger NaCl-Lösung, die 0,05 % Natriumdesoxycholat enthält und 0,012 5 Mol/l an Calciumformiat ist, erneut 30 min lang bei 37 °C gerührt. Dann wird 15 min lang bei 2 500 g zentrifugiert.

Dieser Überstand enthält Thomboplastin ohne proteolytische Aktivität.

Die Aktivität der Thromboplastinsuspension wird mittels Quick-Test, durchgeführt nach DIN 58910, bestimmt. Ein nach DIN 58910 hergestelltes normales Bezugsplasma weist eine Gerinnungszeit von 12 ± 2 sec auf.

B) Herstellung einer Reagensmischung zur Durchführung eines optischen Quick-Tests :

Lösung 1 : 0,1 Mol Tris/HCl, pH 8,1, enthaltend 2 % Glycin

Lösung 2 : wäßrige Lösung von Tos—Gly—Pro—Arg—pNA—Acetat 497 mg/dl

Lösung 3 : Thromboplastinsuspension nach A hergestellt.

Es werden 10 Volumenteile von Lösung 1 mit 4 Volumenteilen Lösung 3 und 1 Volumenteil Lösung 2 gemischt und in 3 ml Aligoten lyophilisiert.

### Beispiel 2

Durchführung eines optischen Quick-Tests

Zur Durchführung des Tests werden 3 ml des Lyophilisats nach Beispiel 1 B in 30 ml Tris/HCl-Puffer, pH 8,1, welcher zusätzlich 1,5 % Harnstoff und 6 mMol/l $CaCl_2$ enthält, gelöst. Das Reaktionsgemisch wird auf 37 °C gebracht. Photometeransatz : Meßtemperatur 37 °C, Wellenlänge 405 nm.

Es werden je 50 µl Probe (Citratplasma, bzw. Verdünnungen von Citratplasma in physiologischem NaCl) vorgelegt und die Reaktion mit 750 µl Reaktionsgemisch gestartet. Gleichzeitig wird ein Schreiber (10 cm/min) gestartet.

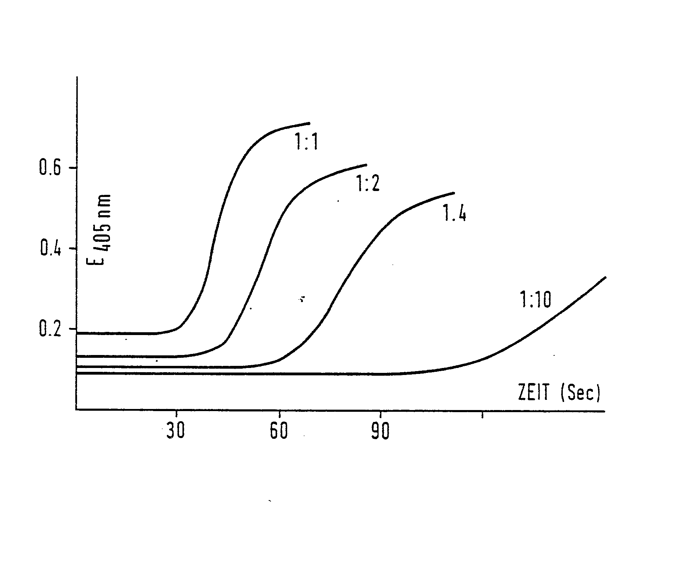

Es werden die in Fig. 1 der beigefügten Zeichnung gezeigten Kurven erhalten, wenn nacheinander unverdünntes bzw. 1 : 2, 1 : 4 und 1 : 10 vorverdünntes Plasma eingesetzt wird.

### Beispiel 3

Aus den nach Beispiel 2 erhaltenen Kurvenzügen wird die Zeit bis zum Erreichen einer gegenüber der Anfangsextinktion um 30 mE höheren Extinktionen entnommen. Diese Zeiten werden gegen die reziproken Plasmaverdünnungen aufgetragen. Man erhält eine Gerade. Die vorgegebene Extinktion von 30 mE kann in weiten Bereichen, z. B. von 10 bis 150 mE, variiert werden. Man erhält stets eine Gerade.

### Beispiel 4

Entsprechend Beispiel 2 werden 60 Plasmen von Patienten, welche unter oraler Antikoagulantien-Therapie stehen, unverdünnt getestet und die Zeit bis zum Erreichen einer um 30 mE gegenüber dem Ausgangswert liegenden Extinktion registriert. Dieselben Plasmen werden mittels des herkömmlichen Quick-Tests unter Verwendung eines Koagulometers nach Schnitker und Gross getestet und die Gerinnungszeiten registriert. Die in beiden Systemen erhaltenen Daten werden einer linearen Regressionsanalyse unterworfen. Es ergibt sich ein Korrelationskoeffizient von r = 0,96.

### Beispiel 5

Entsprechend Beispiel 1 B werden Reagensmischungen unter Verwendung eines nach Beispiel 1 A hergestellten, nicht proteolytischen Thromboplastins sowie eines handelsüblichen Thromboplastins (Thromboplastin a, Boehringer Mannheim) hergestellt. Die Reagensmischungen werden mit Tris/HCl-Puffer, pH 8,1, entsprechend Beispiel 2 zum fertigen Reaktionsgemisch verdünnt und dieses bei Raumtemperatur (ca. 20 °C) gelagert. In bestimmten zeitlichen Abständen wird die Eigenextinktion der Reaktionsgemische bei 405 nm gemessen. Die Ergebnisse zeigt Tabelle 2. Man findet bei dem handelsüblichen Thromboplastin einen raschen Extinktionsanstieg, der auf einer Spaltung des beigefügten chromogenen Peptidsubstrats beruht.

Tabelle 2

| Inkubations-zeit (Tage) | Extinktion bei 405 nm | |
|---|---|---|
| | handelsübliches Thromboplastin | nach Beispiel 1 herge-stelltes Thromboplastin |
| 0 | 0,008 | 0,000 |
| 0,3 | 0,012 | 0,003 |
| 1,0 | 0,028 | 0,010 |
| 2,0 | 0,049 | 0,015 |
| 5,0 | 0,108 | 0,030 |
| 7,0 | 0,154 | 0,035 |

## Patentansprüche

1. Reagenz zur optischen Bestimmung des Blutgerinnungsverhaltens (Quick-Test), enthaltend Thromboplastin, ein synthetisches Thrombinsubstrat und Puffer, gekennzeichnet durch einen Gehalt an einem nicht proteolytisch wirksamem Thromboplastin, welches durch Herstellen eines Acetontrockenpulvers aus Hirn, Inkubation einer neutralen wäßrigen Lösung des Acetontrockenpulvers bei 25 bis 40 °C, Gewinnung der unlöslichen Fraktion, Behandlung einer Suspension dieser Fraktion in verdünnter Salzlösung mit einem oberflächenaktiven Mittel in Gegenwart von Formiat und Trocknung der abgetrennten löslichen Fraktion erhältlich ist.

2. Reagenz nach Anspruch 1, dadurch gekennzeichnet, daß es nicht proteolytisches Thromboplastin, in einer Konzentration von 3 bis 20 Vol.%, $2 \times 10^{-5}$ bis $20 \times 10^{-5}$ Mol/l synthetisches Thrombinsubstrat, 0,05 bis 0,25 Mol/l Puffer pH 7,2 bis 8,5, 2 bis 10 mMol/l lösliches Ca-Salz enthält.

3. Reagenz nach Anspruch 2, dadurch gekennzeichnet, daß es zusätzlich 0,5 bis 3 % Harnstoff enthält.

4. Reagenz nach Anspruch 2 oder 3, dadurch gekennzeichnet, daß es Trispuffer enthält.

## Claims

1. Reagent for the optical determination of the blood coagulation behaviour (Quick test), containing thromboplastin, a synthetic thrombin substrate and buffer, characterised by a content of a non-proteolytically-active thromboplastin which is obtainable by preparation of an acetone dry powder from brain, incubation of a neutral aqueous solution of the acetone dry powder at 25 to 40 °C, obtaining of the insoluble fraction, treatment of a suspension of this fraction in dilute salt solution with a surface-active agent in the presence of formate and drying of the separated soluble fraction.

2. Reagent according to claim 1, characterised in that it contains non-proteolytic thromboplastin in a concentration of 3 to 20 vol.%, $2 \times 10^{-5}$ to $20 \times 10^{-5}$ mol/l. of synthetic thrombin substrate, 0.05 to 0.25 mol/l. of buffer pH 7.2 to 8.5, 2 to 10 mMol/l. of soluble Ca salt.

3. Reagent according to claim 2, characterised in that it additionally contains 0.5 to 3 % of urea.

4. Reagent according to claim 2 or 3, characterised in that it contains tris buffer.

## Revendications

1. Réactif pour la détermination optique du comportement à la coagulation (test de Quick), contenant de la thromboplastine, un substrat de la thrombine synthétique et un tampon, caractérisé en ce qu'il contient une thromboplastine non protéolytiquement active, qui peut être obtenue par préparation d'une poudre de cerveau séchée à l'acétone, incubation d'une solution aqueuse neutre de la poudre séchée à l'acétone à 25 à 40 °C, obtention de la fraction insoluble, traitement d'une suspension de cette fraction dans une solution diluée de sel avec un agent tensio-actif en présence de formiate et séchage de la fraction soluble séparée.

2. Réactif suivant la revendication 1, caractérisé en ce qu'il contient de la thromboplastine non protéolytique à une concentration de 3 à 20 vol.%, $2 \times 10^{-5}$ à $20 \times 10^{-5}$ mole/l de substrat de la thrombine synthétique, 0,05 à 0,25 mole/l de tampon pH 7,2 à 8,5, 2 à 10 mmole/l de sel de Ca soluble.

3. Réactif suivant la revendication 2, caractérisé en ce qu'il contient en outre 0,5 à 3 % d'urée.

4. Réactif suivant la revendication 2 ou 3, caractérisé en ce qu'il contient du tampon tris.